# FASCICULE DE BREVET EUROPEEN

(11) **EP 4 045 016 B1**
(45) Date de publication et mention de la délivrance du brevet: **19.07.2023**
(21) Numéro de dépôt: 21830682.7
(22) Date de dépôt: 13.12.2021
(51) Int. Cl.: A61K 9/20

(54) **COMPRIMÉ À LIBÉRATION PROLONGÉE DE LA MOLSIDOMINE**
MOLSIDOMINTABLETTE MIT VERZÖGERTER FREISETZUNG
SUSTAINED-RELEASE MOLSIDOMINE TABLET

(30) Priorité: 11.12.2020 BE 202005910
(43) Date de publication de la demande: 24.08.2022
(73) Titulaire: Europharmaceuticals, 1180 Uccle (BE)
(72) Inventeur: GECZY, József-Mihály, 1180 Uccle (BE)
(74) Mandataire: Gevers Patents
(86) Numéro de dépôt international: PCT/EP2021/085462
(87) Numéro de publication internationale: WO 2022/123075

(56) Documents cités:
- EP-B1- 0 714 661
- WO-A1-01/62256
- CN-A- 102 341 098

## Description

### Domaine de l'invention

La présente invention concerne un comprimé à libération prolongée comprenant de la molsidomine ou l'un de ses métabolites actifs, un emballage comprenant lesdits comprimés, une composition pharmaceutique comprenant de la molsidomine, ainsi qu'une méthode de production d'un comprimé à libération prolongée comprenant de la molsidomine.

### Arrière-plan de l'invention

La molsidomine (ou N-(éthoxycarbonyl)3-(4-morpholinyl)sydnonimine) est un composé aromatique hétérocyclique mésoionique appartenant à la classe des médicaments nitrovasodilatateurs, c'est-à-dire que son activité biologique est attribuée à sa capacité à libérer le radical NO sous forme gazeuse au cours de sa biotransformation.

Il est connu que la molsidomine est utile dans le traitement préventif de la crise angineuse sous toutes ses formes grâce à sa capacité à provoquer une relaxation de la fibre musculaire lisse vasculaire, ainsi qu'une inhibition des phases précoces de l'activation plaquettaire. Par ailleurs, il a également été démontré que la molsidomine permet de prévenir les processus physiopathologiques conduisant à l'athérosclérose, et d'en ralentir la progression de par sa capacité à restaurer les fonction endothéliales.

La molsidomine a initialement été commercialisée essentiellement sous des formes galéniques nécessitant plusieurs prises quotidiennes. Tout d'abord sous forme de comprimés sécables à libération immédiate dosés à 2 mg et 4 mg, généralement administrés trois fois par jour dans le traitement de l'angor d'effort, et quatre fois par jour dans le traitement de l'angor de repos et de l'effort d'angor sévère. Ensuite, sous forme de comprimés dosés à 8 mg, destinés à être administrés deux fois par jour pour un traitement prophylactique et au long cours de l'angine de poitrine.

Sous ces formes, la concentration plasmatique maximale de la molsidomine est atteinte entre 1 et 3 heures après administration. La molsidomine agit généralement pendant une période de 4 à 5 heures lorsqu'elle est dosée à 4 mg, et pendant une période de 10 à 12 heures, lorsqu'elle est dosée à 8 mg.

De façon générale, il est cependant avantageux de disposer de formes galéniques présentant un effet thérapeutique plus long, permettant de réduire le nombre de prises quotidiennes du médicament. Ceci augment le confort du patient et se traduisant par une meilleure observance de celui-ci à son traitement.

La demande de brevet WO 91/14680 A1 décrit des formes galéniques à libération prolongée de la molsidomine complexée avec des dérivés de la cyclodextrine te destinées à une prise quotidienne unique. Cependant, la préparation des complexes molsidomine-(beta)cyclodextrine nécessite un solvant aqueux, réduisant ainsi la stabilité du principe actif et diminuant, par voie de conséquence, la durée de conservation des compositions pharmaceutiques qui en sont dérivées. Par ailleurs, le fait que l'utilisation de solvants implique au moins une étape de séchage, allongeant de ce fait considérablement le temps nécessaire à la préparation desdits compositions et augmentant les coûts énergétique liés à leur production. Un tel procédé n'est donc pas recommandé pour les principes actifs sensibles à l'hydrolyse, tels que la molsidomine.

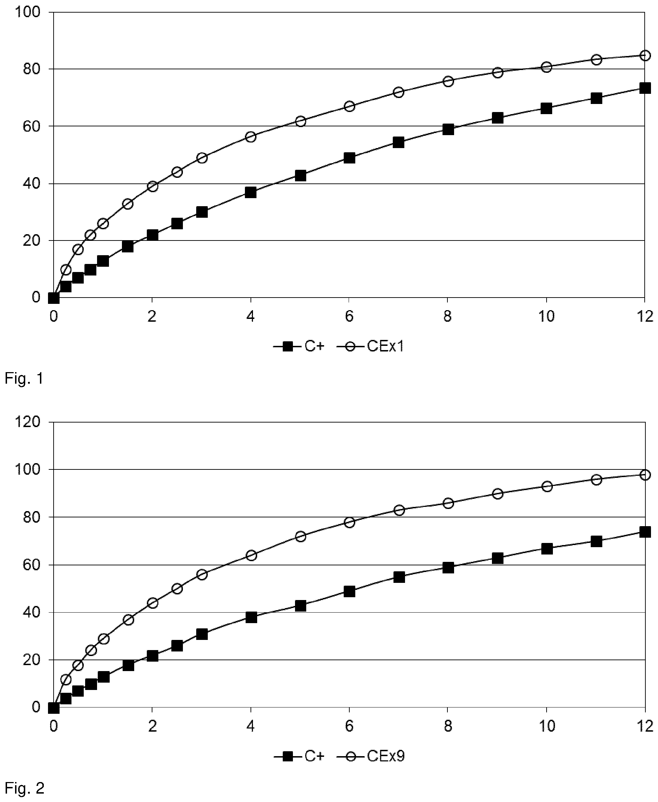

Plus récemment, la molsidomine a été commercialisée (notamment sous la dénomination Coruno^{®} en Belgique) sous la forme d'une composition orale solide à libération prolongée, efficace pendant 24 heures et dosée à 16 mg, pour la prévention et le traitement chronique au long cours de l'angine de poitrine stable. Comme démontré dans la demande de brevet WO 01/62256 A1, sous cette forme galénique la molsidomine fournit une concentration plasmatique pendant environ 24h, contre 4 à 5 heures pour la forme dosée à 4 mg et environ 10 à 12 heures pour le forme dosée à 8 mg (voir en particulier la Figure 1 de WO 01/62256 A1). Par ailleurs, les formulations dosées à 16 mg et décrites dans la demande de brevet WO 01/62256 A1 ont également l'avantage d' entrer rapidement (environ 30 minutes à jeun à 1 h 30 en situation postprandiale) dans la zone thérapeutique (5 à 10 ng/ml) et présentent un pic plasmatique équivalent (33 à 40 ng/ml) à celui mesuré avec les formes galéniques à libération immédiate.

Cependant, de manière à obtenir un tel profil de libération, il s'est avéré que la molsidomine doit être préparée sous la forme de comprimés multicouches, comprenant une couche active, incorporant la molsidomine, intercalée entre deux couches inactives. Or de tels comprimés multicouches présentent un volume supérieur aux comprimés monocouches et sont difficilement sécables, ce qui réduit leur confort d'utilisation. De plus, la production de telles formes galéniques multicouches s'avère contraignante, nécessitant de nombreuse étapes. En effet, chaque couche entrant dans la composition du comprimé doit être granulée et comprimée de manière indépendante. Par ailleurs, chacune desdites couches doit être produite par un procédé de compression dit de « granulation humide », nécessitant une étape d'humectation des mélanges de poudres, suivi d'une étape de séchage, allongeant de ce fait considérablement le temps nécessaire à leur préparation et augment par ailleurs le coût énergétique du procédé.

Une besoin existe donc pour la mise à disposition de formes galéniques de la molsidomine présentant un effet thérapeutique de longue durée ainsi qu'une durée de conservation étendue et pouvant être produites de façon simple et à un coût raisonnable.

### Brève description de l'invention

La présente invention porte sur un comprimé à libération prolongée comprenant de 14 à 24 mg de molsidomine, de 45 à 80 mg de monohydrogénophosphate de calcium dihydraté, de 35 à 60 mg d'un agent matriciel lipophile, de 15 à 45 mg de cellulose microcristalline, de 0.10 à 10 mg de silice colloïdale anhydre, et de 0.50 à 5 mg de stéarate de magnésium, dans lequel ledit comprimé est obtenu par un procédé de compression directe. Parmi les agents matriciels lipophiles, le béhénate de glycérol est préféré. Les excipients présents dans la préparation des comprimés selon l'invention permettent une administration quotidienne unique. Les comprimés sont obtenus par un processus de compression directe, permettant une production rapide et peu coûteuse desdits comprimés toute en maintenant les constituants sous une forme essentiellement sèche.

La présente invention porte également sur une méthode de production d'un comprimé à libération prolongée par un procédé de compression directe comprenant :
- fournir un pré-mélange essentiellement sec comprenant un ou plusieurs excipients pharmacologique ment acceptables,
- mélanger de la molsidomine sous forme essentiellement sèche avec ledit pré-mélange essentiellement sec de manière à obtenir une préparation essentiellement sèche, et
- comprimer ladite préparation essentiellement sèche de manière à obtenir ledit comprimé à libération prolongée.

### Brève description des figures

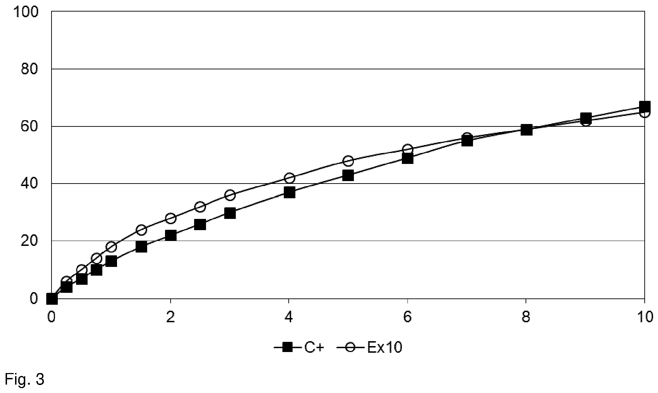

Les figures 1 à 3 représentent la cinétique de libération de la molsidomine des comprimés CEx 1, CEx 9 et Ex 10, respectivement, comparée à un contrôle positif (« C+ »). L'axe vertical représente le pourcentage de dissolution des comprimés (%). L'axe horizontal représente le temps, exprimé en heures.

### Description détaillée de l'invention

Le terme « comprenant », utilisé dans les revendications, ne doit pas être interprété comment étant limité aux moyens énumérés par après ; il n'exclut pas d'autres éléments ou étapes. Il doit être interprété comme précisant la présence des caractéristiques énumérées, les éléments, étapes ou composants auxquels il est fait référence, mais n'exclut pas la présence ou l'ajout d'une ou plusieurs autres caractéristiques, éléments, étapes, ou composants, ou des groupes de ceux-ci. Ainsi, la portée de l'expression « un comprimé comprenant A et B » ne doit pas être limitée au comprimé composé uniquement de A et B. Cela signifie que, dans le respect de la présente invention, les seuls éléments pertinents dans le comprimé sont A et B. En conséquence, le termes « comprenant » et « incluant » englobent les termes plus restrictifs que sont « consistant essentiellement en » et « consistant en ».

L'expression « comprimé à libération prolongée » désigne un comprimé dans lequel la vitesse de libération de la substance active est ralentie, la libération de la substance active étant étalée dans le temps (par opposition à une libération immédiate). De préférence, l'expression « comprimé à libération prolongée » désigne un comprimé assurant la diffusion du principe actif sur une durée plus longue que la libération conventionnelle. Le terme « prolongée » signifie que le principe actif est libéré durant une période d'au moins environ 12 heures, de préférence au moins environ 16 heures, de manière encore plus préférentielle au moins environ 20 heures, avantageusement au moins environ 24 heures.

Le terme « blister » désigne plusieurs types d'emballages préformés utilisés pour les petits biens de consommation, les aliments, et les produits pharmaceutiques. Les blisters sont utiles pour protéger les produits contre les facteurs externes, tels que l'humidité, la lumière ou les contaminations pendant de longues périodes.

L'expression « essentiellement sec » en lien avec une composition signifie que ladite composition ne comprend pas de liquide supplémentaire par rapport à ceux intrinsèquement présents dans les ingrédients qui la compose. Par exemple, une composition comprenant un ingrédient anhydre sera considérée essentiellement sèche, de la même manière qu'une composition comprenant ledit ingrédient sous forme hydratée, pour autant que ladite composition ne comprenne pas d'autres ingrédients sous forme liquide, en particulier pas de solvant liquide.

L'expression « capping » désigne le détachement d'un fragment de la section transversale d'un comprimé, qui se produit juste après l'éjection du comprimé de la matrice. Les dommages causés par le "capping" entrainent le rejet du lot de production.

Comme décrit ci-dessus, la présente invention porte sur un comprimé à libération prolongée comprenant
- de 14.00 à 24.00 mg de molsidomine,
- de 45.00 à 80.00 mg de monohydrogénophosphate de calcium dihydraté,
- de 35.00 à 60.00 mg d'un agent matriciel lipophile,
- de 15.00 à 45.00 mg de cellulose microcristalline,
- de 0.10 à 10.00 mg de silice colloïdale anhydre, et
- de 0.50 à 5.00 mg de stéarate de magnésium, dans lequel ledit comprimé est obtenu par un procédé de compression directe.

Les inventeurs ont découvert que les comprimés selon la présente invention permettent une libération prolongée de la molsidomine sur une durée de 24 heures, offrant de ce fait un grand confort d'utilisation au patient, puisqu'une prise quotidienne unique est suffisante pour obtenir l'effet thérapeutique recherché.

Ensuite, la nouvelle forme galénique a l'avantage de pouvoir se présenter sous une forme monocouche pouvant être produite par un procédé de compression directe, permettant ainsi une production simple à un coût raisonnable desdits comprimés.

Par ailleurs, la production de la nouvelle forme galénique par compression directe peut être effectuée à partir de matières premières sèches, ce qui garantit une conservation sur une période étendue.

La molsidomine est connue pour son efficacité dans le traitement préventif de la crise angineuse sous toutes ses formes, ainsi que pour le traitement de l'athérosclérose des artères coronaires.

Tel que décrit dans le présent document, le terme « molsidomine » désigne aussi bien la forme libre de la molécule que ses sels pharmaceutiquement acceptables, tel qu'en particulier un chlorhydrate.

Par ailleurs, les comprimés selon la présente invention peuvent contenir de la molsidomine ou l'un de ses métabolites actifs. En effet, la molsidomine est une prodrogue. Après prise orale, la molsidomine subit une transformation enzymatique au niveau hépatique produisant ainsi le SIN-1 (linsidomine). Le SIN-1 est-lui-même transformé rapidement au niveau sanguin et sans intervention enzymatique en SIN-1A. Le SIN-1A est enfin dégradé par oxydation en SIN-1C inactif avec libération de monoxyde d'azote (NO), lequel monoxyde d'azote étant le véritable composé vasodilatateur. Ainsi, les dérivés actifs de la molsidomine tels que visés par l'expression « molsidomine ou l'un de ses dérivés actifs » dans le cadre de la présente invention désigne préférentiellement le SIN-1 et le SIN-1A. Selon une variante avantageuse de l'invention, la molsidomine ou l'un de ses métabolites actifs est la molsidomine.

Comme décrit ci-dessus, la quantité de molsidomine ou l'un de ses métabolites comprise dans le comprimé à libération prolongée varie entre 14.00 et 24.00 mg. Le comprimé à libération prolongée selon l'invention comprend de 14.00 à 24.00 mg de molsidomine de préférence de 16.00 à 20.00 mg de molsidomine et de manière encore plus préférée environ 16 mg de molsidomine.

Selon une variante préférée de l'invention, la molsidomine comprise dans le comprimé à libération prolongée n'est pas complexée avec un dérivé de la cyclodextrine. En particulier, la molsidomine n'est pas comprise dans un complexe d'inclusion formé avec l'heptakis-2,6-0-dimethyl-β-cyclodextrine (Dimed), l'hydroxypropyl- β-cyclodextrine ou avec la β- ou γ-cyclodextrine. En effet, la préparation des complexes entre la molsidomine et un dérivé de la cyclodextrine nécessite un solvant aqueux, réduisant ainsi la stabilité du principe actif et diminuant, par voie de conséquence, la durée de conservation des compositions pharmaceutiques qui en sont dérivées. En revanche, les comprimés non-complexés avec un dérivé de la cyclodextrine présentent une stabilité excellente durant une période d'au moins 24 mois (voir Tableau 4). Par ailleurs, le fait que l'utilisation de solvants implique au moins une étape de séchage, allongeant de ce fait considérablement le temps nécessaire à la préparation desdits compositions et augmentant les coûts énergétique liés à leur production.

Le comprimé à libération prolongée selon la présente invention comprend par ailleurs du monohydrogénophosphate de calcium. Le terme « monohydrogénophosphate de calcium » désigne aussi bien les formes hydratées, que les formes anhydres du monohydrogénophosphate de calcium. Selon une variante avantageuse de l'invention, le comprimé à libération prolongée comprend la forme dihydratée du monohydrogénophosphate de calcium. En effet, la forme dihydratée du monohydrogénophosphate de calcium présente de bonnes propriétés découlement et une faible hygroscopie. De préférence, le monohydrogénophosphate de calcium compris dans le comprimé à libération prolongée est le produit commercial EMCOMPRESS^{®} Premium dihydrate (DCP dihydrate).

Comme indiqué, la quantité de monohydrogénophosphate de calcium comprise dans le comprimé à libération prolongée varie entre 45.00 et 80.00 mg. De préférence, le comprimé comprend entre 50.00 et 70.00 mg dudit monohydrogénophosphate de calcium, plus avantageusement entre 55.00 et 65.00 mg dudit monohydrogénophosphate de calcium, encore plus avantageusement environ 57 mg dudit monohydrogénophosphate de calcium.

Le comprimé à libération prolongée selon la présente invention comprend en outre un agent matriciel lipophile. L'expression « agent matriciel lipophile » tel qu'utilisée dans la présente description peut être comprise dans son sens le plus large, et fait référence à une substance ayant la capacité de se dissoudre dans les graisses, huiles, lipides et les solvant apolaires (tels que l'hexane ou le toluène), et pouvant être utilisé comme matrice chimiquement inactive dans la formulation d'un médicament, un cosmétique ou un aliment. De manière avantageuse, l'agent matriciel lipophile selon la présente invention est non-seulement insoluble dans l'eau, mais par ailleurs ne gonfle pas et ne s'érode pas au contact de l'eau. De cette manière, la cinétique de libération du principe actif contenu dans les comprimés est principalement du premier ordre. Dans une variante du comprimé à libération prolongée selon l'invention, l'agent matriciel lipophile peut être choisi parmi les huiles de ricin hydrogénées (Curtina^{®}), les alcools stéarilique, cétostéarilique, cétylique, des mono-, di-, triglycérides, seuls ou en combinaison avec des cires, de la paraffine, des céramides, des dérivés du cholestérol ou d'autres lipides apolaires. De préférence, l'agent matriciel lipophile est un mono-, di- ou trigycéride, ou un mélange de ceux-ci, de manière encore plus préférentielle l'agent matriciel lipophile est un mélange de mono-, di- et triglycérides d'acide béhénique (C22:0). Avantageusement, on utilisera dans le cadre de la présente invention un béhénate de glycérol, tel que le produit connu sous la dénomination commercial Compritol^{®} 888 ATO.

Comme indiqué, la quantité d'agent matriciel lipophile comprise dans le comprimé à libération prolongée varie entre 35.00 et 60.00 mg. De préférence, le comprimé comprend entre 38.00 et 55.00 mg dudit agent matriciel lipophile, plus avantageusement entre 40.00 et 50.00 mg dudit agent matriciel lipophile, encore plus avantageusement environ 45 mg dudit agent matriciel lipophile.

Selon une variante préférée de l'invention, le comprimé à libération prolongée de la présent invention comprend entre 35.00 et 60.00 mg d'un béhénate de glycérol. De préférence, le comprimé comprend entre 38.00 et 55.00 mg dudit béhénate de glycérol, plus avantageusement entre 40.00 et 50.00 mg dudit béhénate de glycérol, encore plus avantageusement environ 45 mg dudit béhénate de glycérol.

De manière avantageuse, le comprimé à libération prolongée de la présente invention comprendra en outre de 15.00 à 45.00 mg d'un agent liant sec, qui est de la cellulose microcristalline. L'agent liant sec selon l'invention est destiné à agir comme un adhésif afin de lier ensemble les autres ingrédients pour conférer au comprimé la résistance mécanique nécessaire. Un tel agent liant sec est avantageusement une cellulose microcristalline. De préférence, la cellulose microcristalline selon l'invention est telle que au moins environ 90% des particules de cellulose ont une taille inférieure à 200 µm. De manière encore plus préférentielle, la taille moyenne des particules de cellulose microcristalline est d'environ 100 µm. Avantageusement, on utilisera dans le cadre de la présente invention une cellulose microcristalline telle que le produit connu sous la dénomination commerciale AVICEL^{®} PH 102. De préférence, le comprimé à libération prolongée comprend de 20.00 à 40.00 mg dudit agent liant sec, plus avantageusement de 25.00 à 35.00 mg, encore plus avantageusement environ 30 mg dudit agent liant sec.

Selon une variante préférée de l'invention, le comprimé à libération prolongée de la présent invention comprend entre 20.00 et 40.00 mg de cellulose microcristalline, plus avantageusement de 25.00 à 35.00 mg, encore plus avantageusement environ 30 mg de ladite cellulose microcristalline.

Le comprimé à libération prolongée selon la présente invention comprendra en outre généralement de 0.10 à 10.00 mg d'un agent régulateur d'écoulement qui est la silice colloïdale anhydre. De tels agents régulateurs d'écoulement sont connus de l'homme du métier et sont utilisés pour favoriser l'écoulement des poudres en réduisant la friction et la cohésion entre les particules. Un tel agent régulateur d'écoulement est de préférence la silice colloïdale, plus avantageusement la silice colloïdale anhydre, telle que le produit connu sous le nom commercial AEROSIL^{®} 200. De préférence, le comprimé à libération prolongée comprend de 0.20 à 5.00 mg dudit agent régulateur d'écoulement, plus avantageusement de 0.30 à 1.00 mg, encore plus avantageusement de 0.40 à 0.60 mg, voire il comprend environ 0.5 mg dudit agent régulateur d'écoulement.

Selon une variante préférée de l'invention, le comprimé à libération prolongée de la présent invention comprend entre 0.20 et 5.00 mg de silice colloïdale, plus avantageusement de 0.30 à 1.00 mg, encore plus avantageusement de 0.40 à 0.60 mg, voire comprend environ 0.5 mg de ladite silice colloïdale, ladite silice colloïdale étant de préférence la silice colloïdale anhydre.

De manière avantageuse, le comprimé à libération prolongée selon la présente invention comprendra en outre de 0.50 à 5.00 mg d'un agent lubrifiant qui est le stéarate de magnésium. Typiquement, les agents lubrifiants permettent d'empêcher les ingrédients de s'agglutiner et de coller lors de la production des comprimés. De manière générale, les agents lubrifiants améliorent la fabricabilité des comprimés. De tels agents lubrifiants sont par exemple le talc, la silice, les graisses, telle que la stéarine végétale, le stéarate de magnésium ou l'acide stéarique. De préférence, l'agent lubrifiant est le stéarate de magnésium. Avantageusement, le comprimé à libération prolongée comprend de 0.75 à 3.00 mg dudit agent lubrifiant, de manière encore plus avantageuse de 1.00 à 2.00 mg, encore plus avantageusement environ 1.5 mg dudit agent lubrifiant.

Selon une variante préférée de l'invention, le comprimé à libération prolongée de la présent invention comprend entre 0.75 et 3.00 mg de stéarate de magnésium, plus avantageusement de 1.00 à 2.00 mg, encore plus avantageusement environ 1.5 mg de stéarate de magnésium.

De manière avantageuse, le comprimé à libération prolongée selon la présente invention est substantiellement exempt de dérivés de la cyclodextrine. Dans le contexte de la présente invention, l'expression « substantiellement exempt de dérivés de la cyclodextrine » est destinée à signifier qu'aucun dérivé de la cyclodextrine n'est présent dans le comprimé à libération prolongée, ou uniquement quelques traces non-détectables par des techniques classiques, notamment des traces inférieures à 5 parties par million (ppm), plus particulièrement inférieures à 1 ppm de dérivés de la cyclodextrine. En particulier, le comprimé à libération prolongée est substantiellement exempt de heptakis-2,6-0-dimethyl-β-cyclodextrine (Dimed), de hydroxypropyl- β-cyclodextrine et/ou de β- ou γ-cyclodextrine.

De manière avantageuse de la présente invention, le comprimé à libération prolongée comprend de 14.00 à 24.00 mg de molsidomine, de 45.00 à 80.00 mg de monohydrogénophosphate de calcium dihydraté, de 35.00 à 60.00 mg d'un agent matriciel lipophile, de préférence un béhénate de glycérol, de 15.00 à 45.00 mg de cellulose microcristalline, de 0.10 à 10.00 mg de silice colloïdale anhydre et 0.50 à 5.00 mg de stéarate de magnésium. Encore plus avantageusement, le comprimé à libération prolongée selon la présente invention comprend de 16.00 à 20.00 mg de molsidomine, de 55.00 à 65.00 mg de monohydrogénophosphate de calcium dihydraté, de 40.00 à 50.00 mg d'un agent matriciel lipophile, de préférence un béhénate de glycérol, de 25.00 à 35.00 mg de cellulose microcristalline, de 0.40 à 0.60 mg de silice colloïdale anhydre et 1.00 à 2.00 mg de stéarate de magnésium.

Selon une variante préférée de l'invention, le comprimé à libération prolongée comprend 16 mg de molsidomine, 30 mg de monohydrogénophosphate de calcium dihydraté, 45 mg de béhénate de glycérol, 30 mg de cellulose microcristalline, 0.5 mg de silice colloïdale anhydre et 1.5 mg de stéarate de magnésium.

Par ailleurs, le comprimé à libération prolongée selon l'invention peut contenir d'autres excipients. Par exemple, le comprimé à libération prolongée peut contenir des arômes, des édulcorants, des exhausteurs de goût, des colorants alimentaires ainsi que des stabilisateurs. Cependant, de manière avantageuse, le comprimé à libération prolongée selon l'invention ne comprend pas d'autres excipients que ceux cités ci-dessus.

La présente invention porte en outre sur un emballage comprenant les comprimés à libération prolongée de l'invention, lesdits comprimés étant conditionnés dans au moins un blister, ledit blister étant lui-même emballé dans ledit emballage. De préférence, ledit emballage comprend entre 28 et 62 comprimés à libération prolongée. Par ailleurs, ledit blister permet avantageusement de protéger lesdits comprimés à libération prolongée de la lumière, de manière à accroitre leur durée de conservation. En effet, la molsidomine est un composé particulièrement sensible à la lumière, et aux rayons UV en particulier.

La forme galénique mise au point par les inventeurs permet une libération prolongée et calibrée de molsidomine ou l'un de ses métabolites actifs permettant une seule prise quotidienne et assurant ainsi une meilleure compliance du patient. De manière importante, il a été découvert que l'allongement de la couverture thérapeutique peut être obtenu sans diminution significative de la concentration plasmatique maximale, avec un entrée rapide dans la zone thérapeutique. Ainsi, le comprimé à libération prolongée selon l'invention libère une proportion suffisante et calibrée de molsidomine ou l'un de ses métabolites actifs en milieu acide, et donc principalement dans l'estomac, ce qui assure une entrée rapide (environ 30 min à jeun, à 1h30 en situation postprandiale) dans la zone thérapeutique (5 à 10 ng/ml) et un pic plasmatique équivalent (33 à 40 ng/ml) à celui mesuré avec les formes galéniques à libération immédiate.

Ainsi, le comprimé à libération prolongée selon l'invention présente avantageusement un taux de dissolution *in vitro* tel que entre 40 et 60% en poids de la molsidomine est libérée après 6 heures et moins de 80% en poids de la molsidomine est libérée après 10 heures. Aux fins de la présente invention, le taux de dissolution *in vitro* est mesuré spectrophotométriquement à 310 nm selon la méthode décrite dans la Pharmacopée Européenne, 3ème édition (ou U.S.P. XXIV), à 50 t.p.m. dans 500 mL d'un tampon phosphate 0.05 M, pH 6.8, à 37°C.

Par ailleurs, le comprimé à libération prolongée selon l'invention est de préférence caractérisé par un pic plasmatique *in vivo* se présentant dans les 2.5 à 5 heures, de préférence dans les 3 à 4 heures, suivant l'administration dudit comprimé et ayant une valeur comprise entre 25 et 40 ng/ml de plasma. L'expression « pic plasmatique *in vivo* » correspond à la concentration maximale moyenne de molsidomine trouvée dans le plasma d'au moins 10 volontaires en bonne santé. Par ailleurs, l'expression « quantité thérapeutique ment efficace » signifie dans le cadre de la présente invention une quantité de molsidomine suffisante pour fournir une concentration plasmatique d'au moins 5 ng/ml, et de préférence d'au moins 10 ng/ml de plasma, pendant une période d'environ 24 heures.

Ainsi, le comprimé à libération prolongée selon l'invention peut être administré oralement une fois par jour. La présente invention porte donc en outre sur un méthode de traitement comprenant l'administration d'un comprimé à libération prolongée selon l'invention pour utilisation dans un traitement, ledit traitement comprenant l'administration d'un seul comprimé à libération prolongée par jour.

Le comprimé à libération prolongée selon l'invention est destiné à être utilisé comme médicament, plus précisément destiné au traitement et/ou à la prévention de l'angine de poitrine sous toutes ses formes (angor d'effort ou de repos, angor instable), ainsi qu'au traitement et/ou à la prévention de l'athérosclérose, de préférence l'athérosclérose coronaire.

Par ailleurs, il est également avantageux que le comprimé à libération prolongée puisse être obtenu de manière simple et peu coûteuse. Ainsi, le comprimé à libération prolongée selon la présente invention peut être obtenu par un procédé de compression directe.

La présente invention porte donc en outre sur une méthode de production d'un comprimé à libération prolongée de l'invention, comprenant
- fournir un pré-mélange essentiellement sec comprenant un ou plusieurs excipients pharmaceutiquement acceptables,
- mélanger de la molsidomine sous forme essentiellement sèche avec ledit pré-mélange essentiellement sec de manière à obtenir un préparation essentiellement sèche, et
- comprimer ladite préparation essentiellement sèche de manière à obtenir ledit comprimé à libération prolongée.

De préférence, ledit pré-mélange essentiellement sec comprend du monohydrogénophosphate de calcium, de manière plus préférée du monohydrogénophosphate de calcium dihydraté. Dans une variante avantageuse de la méthode de production, ledit pré-mélange comprend en outre de la cellulose microcristalline et/ou de la silice colloïdale, de préférence de la silice colloïdale anhydre.

Par ailleurs, de préférence, ladite méthode de production comprend en outre l'ajout d'un agent matriciel lipophile, avantageusement un béhénate de glycérol, avant l'étape de compression. De manière particulièrement avantageuse, ladite méthode de production comprend en outre une étape dans laquelle du stéarate de magnésium est ajouté à ladite préparation essentiellement sèche de manière à faciliter sa compression.

Selon une variante préférée de la présente méthode de production, le comprimé à libération prolongée selon la présente invention est produit par un procédé de compression directe. Avantageusement, ledit procédé comprend :
- fournir un pré-mélange essentiellement sec comprenant du monohydrogénophosphate de calcium dihydraté, de la cellulose microcristalline et de la silice colloïdale anhydre,
- mélanger de la molsidomine sous forme essentiellement sèche et un agent matriciel lipophile, de préférence un béhénate de glycérol, avec ledit pré-mélange essentiellement sec, de manière à obtenir une préparation essentiellement sèche, et
- comprimer ladite préparation essentiellement sèche de manière à obtenir ledit comprimé à libération prolongée.

L'expression « procédé de compression directe » désigne une procédé de production de comprimés pharmaceutiques consistant en la compression d'un mélange de poudres composé d'un ou plusieurs principe(s) actif(s) et excipient(s). Ce procédé se définit par opposition aux procédés dits de « granulation humide » et de « granulation sèche », impliquant tous deux une étape supplémentaire de densification menant à la formation de granulés (secs ou humides) qui seront ensuite comprimés pour obtenir le comprimé pharmaceutique.

Dans une variante avantageuse, ladite méthode de production comprend en outre une étape dans laquelle lesdits comprimés à libération prolongée sont conditionnés, de préférence dans un blister.

La présente invention concerne en outre une composition pharmaceutique comprenant de la molsidomine, ladite composition pharmaceutique pouvant être comprimée par un processus de compression directe de manière à obtenir le comprimé à libération prolongée de l'invention. Ladite composition pharmaceutique comprendra donc de manière avantageuse, exprimé en ratio pondéral relatif au poids de molsidomine présente dans la composition
- entre 2 : 1 et 5 : 1 de monohydrogénophosphate de calcium, de préférence de monohydrogénophosphate de calcium dihydraté,
- entre 1.5 : 1 et 3 : 1 de cellulose microcristalline,
- entre 1 : 10 et 1 : 50 de silice colloïdale, de préférence de silice colloïdale anhydre,
- entre 2 : 1 et 5 : 1 d'un béhénate de glycérol, et
- entre 1 : 5 et 1 : 20 de stéarate de magnésium.

Selon une variante avantageuse de l'invention, ladite composition pharmaceutique comprendra donc de manière avantageuse, exprimé en ratio pondéral relatif au poids de molsidomine présente dans la composition
- entre 3 : 1 et 4 : 1 de monohydrogénophosphate de calcium, de préférence de monohydrogénophosphate de calcium dihydraté,
- entre 1.7 : 1 et 2 : 1 de cellulose microcristalline,
- entre 1 : 20 et 1 : 40 de silice colloïdale, de préférence de silice colloïdale anhydre,
- entre 2.5 : 1 et 3 : 1 d'un béhénate de glycérol, et
- entre 1 : 8 et 1 : 12 de stéarate de magnésium.

L'invention va maintenant être décrite plus en détail en se référant aux exemples suivants, qui détaillent des illustrations non-limitatives de différents aspects de l'invention.

### Exemples

### Ingrédients

**Tableau 1 - ingrédients de base utilisés dans la préparation des comprimés décrits ci-après**

| **Ingrédient** | **Nom commercial** |
|---|---|
| Molsidomine | / |
| Lactose monohydrate | Lactose DCL 15 |
| Lactose monohydrate :cellulose (75 : 25) | CELLACTOSE^{®} 80 |
| Monohydrogénophosphate de calcium dihydraté | EMCOMRPESS^{®} Premium dihydraté |
| Cellulose microcristalline 100µm | AVICEL^{®} PH 102 |
| Cellulose microcristalline 180µm | AVICEL^{®} PH 200 |
| Silice colloïdal anhydre | AEROSIL^{®} 200 |
| Huile de ricin hydrogénée | CUTINA^{®} HR |
| Béhénate de glycérol | COMPRITOL^{®} ATO 888 |
| Stéarate de magnésium | / |

### 1. Préparation des comprimés

### Exemples comparatifs 1 - 9 (CEx 1-9) et Exemple 10 (Ex 10)

Les comprimés ont été préparés par un procédé de compression directe en l'absence d'un solvant. L'agent diluant, l'agent liant sec ainsi que l'agent régulateur d'écoulement ont été mélangés intimement à l'aide d'un mélangeur TURBULA^{®} durant 2 minutes. La poudre ainsi obtenue a ensuite été tamisée sur un filtre avec un taille de maille moyenne de 800 microns de manière à casser les agrégats. A ce pré-mélange ont ensuite été ajoutées la molsidomine ainsi que l'agent matriciel lipophile, préalablement tamisé. La préparation ainsi obtenue a été mélangée durant 10 minutes à l'aide d'un mélangeur TURBULA^{®}, après quoi l'agent lubrifiant a été ajouté. Après l'ajout de l'agent lubrifiant, et une minute supplémentaire de mélangeage, la masse homogène obtenue a été soumise à une compression de manière à obtenir des comprimés de 7 mm de diamètre, pesant 150 mg chacun.

Les caractéristiques des comprimés des exemples comparatifs 1 - 9 et de l'exemple 10 sont présentées dans le Tableau 2.

**Tableau 2 - Aperçu des caractéristiques des comprimés des exemples comparatifs 1 - 9 et de l'exemple 10**

| **Quantité par comprimé (mg)** | | | **CE x 1** | **CE x 2** | **CE x 3** | **CE x 4** | **CEx 5** | **CE x 6** | **CE x 7** | **CE x 8** | **CE x 9** | **Ex 10** |
|---|---|---|---|---|---|---|---|---|---|---|---|---|
| **Fonction** | **Ingrédient** | | | | | | | | | | | |
| Substance active | Molsidomine | | 16. 0 | 16. 0 | 16. 0 | 16. 0 | 16.0 | 16. 0 | 16. 0 | 16. 0 | 16. 0 | 16. 0 |
| Agent diluant | Lactose monohydrate | | 57. 0 | 49. 5 | 49. 5 | 49. 5 | 49.5 | 49. 5 | 49. 5 | - | 57. 0 | - |
| | Lactose monohydrate:cellulose (75:25) | | - | - | - | - | - | - | - | 87. 0 | - | - |
| | Monohydrogénophosp hate de calcium dihydraté | | - | - | - | - | - | - | - | - | - | 57. 0 |
| Agent liant sec | Cellulose microcristallin e | 100 µm | 30. 0 | 30. 0 | 87. 0 | 94. 5 | 102. 0 | 37. 5 | - | - | 30. 0 | 30. 0 |
| | | 180 µm | - | - | - | - | - | | 37. 5 | - | - | - |
| Agent régulateur d'écouleme nt | Silice colloïdale anhydre | | 0.5 | 0.5 | 0.5 | 0.5 | 0.5 | 0.5 | 0.5 | 0.5 | 0.5 | 0.5 |
| Agent matriciel lipophile | Huile de ricin hydrogénée | | 45. 0 | 52. 5 | 45. 0 | 37. 5 | 30.0 | 45. 0 | 45. 0 | 45. 0 | - | - |
| | Béhénate de glycérol | | - | - | - | - | - | - | - | - | 45. 0 | 45. 0 |
| Agent lubrifiant | Stéarate de magnésium | | 1.5 | 1.5 | 1.5 | 1.5 | 1.5 | 1.5 | 1.5 | 1.5 | 1.5 | 1.5 |
| Total | | | 150 | 150 | 150 | 150 | 150 | 150 | 150 | 150 | 150 | 150 |

### 2. Analyse de la régularité de poids et de la résistance mécanique des comprimés

Les comprimés des exemples comparatifs 1-9 et de l'exemple 10 ont été comprimés en utilisant 3 forces de compression : 80 kg au manomètre (P1), 60 kg au manomètre (P2) et 40 kg au manomètre (P3). Pour chacun des comprimés testés, la régularité du poids ainsi que la résistance mécanique (dureté) ont été évalués pour chacune des 3 forces de compression, mis à part pour les comprimés de exemples comparatifs CEx 6-8, où ces paramètres n'ont été évalués que pour la force de compression P1. Le test de dureté a été effectué à l'aide d'un système de dureté SOTAX^{®}. Les résultats de cette analyse sont résumés dans le Tableau 3.

**Tableau 3 - Analyse de la régularité du poids et de la résistance mécanique des comprimés des exemples comparatifs 1 - 9 et de l'exemple 10 obtenus aux forces de compression P1, P2 et P3**

| | **Poids moyen (mg)** | | | **Dureté moyenne (N)** | | |
|---|---|---|---|---|---|---|
| **Force de compression** | **P1** | **P2** | **P3** | **P1** | **P2** | **P3** |
| **CEx 1** | 150.4±1.6 | 150.4±1.6 | 150.4±1.6 | 66.9±5.8 | 63.9±5.3 | 60.5±5.0 |
| **CEx 2** | 151.5±17.0 | 137.9±23.1 | 140.6±28.6 | n.a. | n.a. | n.a. |
| **CEx 3** | n.a. | n.a. | n.a. | n.a. | n.a. | n.a. |
| **CEx 4** | n.a. | n.a. | n.a. | n.a. | n.a. | n.a. |
| **CEx 5** | n.a. | n.a. | n.a. | n.a. | n.a. | n.a. |
| **CEx 6** | 150.4±2.8 | - | - | 73.8±4.76 | - | - |
| **CEx 7** | 149.3±4.4 | - | - | 66.7±4.69 | - | - |
| **CEx 8** | 150.9±2.4 | - | - | 59.2±14.9 | - | - |
| **CEx 9** | 152.6±0.9 | 153.0±1.7 | 151.2±1.3 | 59.3±4.6 | 61.6±2.7 | 55.6±6.1 |
| **EX 10** | 147.78±1.42 | 149.93±1.51 | 149.73±2.04 | 51.0±2.5 | 51.4±1.6 | 51.7±1.8 |

### Résultats

Il ressort de ces analyses que les comprimés CEx 2-5 et CEx 6-8 ne présentent pas les propriétés pharmacotechniques requises pour une commercialisation à grande échelle. En effet, les comprimés CEx 2 présentent un poids très irrégulier, et leur dureté n'a pas pu être évaluée à cause d'un écoulement très peu satisfaisant des poudres qui les constituent. En ce qui concerne les comprimés CEx 3-5, un phénomène de « capping » a été observé lors de la compression, rendant impossible la mesure de la dureté et du poids moyen de ces comprimés. Les comprimés CEx 7 présentent quant à eux un poids trop irrégulier, l'écart-type étant de 4.4 mg, et la coefficient de variation de 2.95%. Enfin, les comprimés CEx 6 et CEx 8 ont également présenté un phénomène de « capping » lors de la compression, ce qui se traduit par un écart-type important lors de l'essai de dureté. Lors d'essais d'effritement subséquents plusieurs comprimés se sont rapidement brisés, confirmant ce phénomène de « capping ».

### 3. Détermination de la dissolution in vitro des comprimés

La cinétique de libération de la molsidomine des comprimés présentant des données de régularité de poids et de dureté acceptables a été évaluée. Ceci concerne donc les comprimés des exemples comparatifs 1 et 9 ainsi que de l'exemple 10. A titre de contrôle positif, un comprimé correspondant à la spécialité pharmaceutique Coruno^{®} commercialisée en Belgique a été utilisé (« C+ »). Cette forme galénique comprenant 16 mg de molsidomine présente des cinétiques de libération *in vitro* et *in vivo* particulièrement avantageuses, décrites notamment dans la demande de brevet internationale WO 01/62256 incorporée ici par référence.

La cinétique de dissolution des comprimés CEx1, CEx 9 et Ex 10 (force de compression de 80 kg au manomètre (P1)) a été évaluée à l'aide d'un appareil Sotax AT 7 équipé de pales, à une vitesse de rotation de 50 rpm, une température de 37°C et dans un milieu de 500 ml composé de tampon phosphate 0.05 M, pH 6.8. Le dosage de la molsidomine libérée était suivi par échantillonnage périodique et mesure par spectrophotométrie UV à 310 nm à l'aide d'un spectrophotomètre HITACHI^{®} U-3000 avec cellule en quartz de 0.3 cm.

### Résultats

Les résultats obtenus sont présentés aux Figures 1 à 3. Bien que les comprimés CEx 1 et CEx 9 donnent des résultats satisfaisants sur le plan technologique (régularité de poids, résistance mécanique - voir Tableau 3, ci-dessus), sur le plan biopharmaceutique, leur cinétique de dissolution est celle de la formule de référence (« C+ »). Les comprimés selon l'Exemple 10, conformes à l'invention, présentent quant à eux une cinétique de dissolution *in vitro* comparable à celle de la formule de référence.

### 4. Détermination de la stabilité des comprimés

La stabilité des comprimés selon l'invention (Exemple 10) a été évaluée sur une période 36 mois. A cet effet, les quantités d'impuretés (produits de dégradation) ainsi que la quantité de principe actif (molsidomine intacte) ont été dosées à intervalles réguliers. Parmi les impuretés détectées, la nitrosomorpholine est le produit de dégradation majoritaire de la molsidomine., sa quantité devant demeurer inférieure ou égale à 15 ppm au terme de la durée de validité du comprimé. La stabilité des comprimés a été évaluée en conditions de conservation normales (25 °C et 60 % d'humidité relative).

### Résultats

Les résultats obtenus sont présentés dans le Tableau 4, ci-dessous. L'expression « < L.D. » signifie que la quantité d'impuretés se trouve sous la limite de détection de la méthode analytique. Les comprimés selon l'Exemple 10, conformes à l'invention, présentent une quantité de molsidomine conforme ( > 95 %) sur l'ensemble de la durée de l'étude de stabilité, soit 36 mois. Par ailleurs, la quantité d'impuretés, et en particulier la quantité de nitrosomorpholine, reste conforme (au plus 15 ppm pour la nitrosomorpholine) durant une période d'au moins 24 mois. Les comprimés selon l'invention présentent donc une excellente stabilité sur une période d'au moins 24 mois.

**Tableau 4 - Analyse de la stabilité des comprimés de l'Exemple 10**

| *Durée de conservation (mois)* | **Dosage de la Molsidomine** (%) | **Dosage des impuretés** | |
|---|---|---|---|
| | | **Totales** (%) | **Nitrosomorpholine (ppm)** |
| 0 | 97,8 | < L.D. | < L.D. |
| 3 | 100,1 | 0,02 | < L.D. |
| 6 | 95,8 | 0,04 | < L.D. |
| 9 | 98,2 | 0,05 | < L.D. |
| 12 | 98,2 | - | 7-8 |
| 18 | 101,6 | 0,09 | 9-10 |
| 24 | 97,9 | - | 15 |
| 36 | 98,7 | 0,13 | 23 |

## Revendications

1. Comprimé à libération prolongée comprenant :
- de 14.00 à 24.00 mg de molsidomine,
- de 45.00 à 80.00 mg de monohydrogénophosphate de calcium dihydraté,
- de 35.00 à 60.00 mg d'un agent matriciel lipophile,
- de 15.00 à 45.00 mg de cellulose microcristalline,
- de 0.10 à 10.00 mg de silice colloïdale anhydre, et
- de 0.50 à 5.00 mg de stéarate de magnésium,
dans lequel ledit comprimé est obtenu par un procédé de compression directe.

2. Comprimé à libération prolongée selon la revendication 1, dans lequel ledit comprimé présente un taux de libération *in vitro* tel que entre 40 et 60 % en poids de la molsidomine est libérée après 6 heures et moins de 80 % en poids de la molsidomine est libérée après 10 heures.

3. Comprimé à libération prolongée selon l'une quelconque des revendications précédentes pour utilisation comme médicament.

4. Comprimé à libération prolongée selon la revendication 3 pour utilisation dans le traitement et/ou la prévention de l'angine de poitrine et/ou de l'athérosclérose coronaire, optionnellement où le comprimé est administré une fois par jour.

5. Emballage comprenant entre 28 et 62 comprimés à libération prolongée selon les revendications 1 et 2, lesdits comprimés à libération prolongée étant conditionnés dans au moins un blister protégeant lesdits comprimés à libération prolongée de la lumière, ledit blister étant lui-même emballé dans ledit emballage.

6. Méthode de production d'un comprimé à libération prolongée selon l'une quelconque des revendications 1 et 2 comprenant:
- fournir un pré-mélange essentiellement sec comprenant un ou plusieurs excipients pharmaceutiquement acceptables,
- mélanger de la molsidomine sous forme essentiellement sèche avec ledit pré-mélange essentiellement sec de manière à obtenir une préparation essentiellement sèche, et
- comprimer ladite préparation essentiellement sèche de manière à obtenir ledit comprimé à libération prolongée.

7. Méthode selon la revendication 6, comprenant :
- fournir un pré-mélange essentiellement sec comprenant du monohydrogénophosphate de calcium dihydraté, de la cellulose microcristalline et de la silice colloïdale anhydre,
- mélanger de la molsidomine sous forme essentiellement sèche et d'un agent matriciel lipophile avec ledit pré-mélange essentiellement sec de manière à obtenir une préparation essentiellement sèche,
- comprimer ladite préparation essentiellement sèche de manière à obtenir ledit comprimé à libération prolongée,
- et optionnellement conditionner ledit comprimé à libération prolongée, de préférence dans un blister protégeant lesdits comprimés à libération prolongée de la lumière.

## Patentansprüche

1. Tablette mit verzögerter Freisetzung, umfassend:
- von 14,00 bis 24,00 mg an Molsidomin,
- von 45,00 bis 80,00 mg an Calcium-Dihydrat-Monohydrogenphosphat,
- von 35,00 bis 60,00 mg eines lipophilen Matrixmittels,
- von 15,00 bis 45,00 mg an mikrokristalliner Cellulose,
- von 0,10 bis 10,00 mg an wasserfreier kolloidaler Kieselsäure, und
- von 0,50 bis 5,00 mg an Magnesiumstearat,
wobei die Tablette durch ein direktes Kompressionsverfahren erhalten wird.

2. Tablette mit verzögerter Freisetzung nach Anspruch 1, wobei die Tablette eine in Vitro Freisetzungsrate aufweist, sodass zwischen 40 und 60 Gew.-% an Molsidomin nach 6 Stunden freigesetzt werden und weniger als 80 Gew.-% an Molsidomin nach 10 Stunden freigesetzt werden.

3. Tablette mit verzögerter Freisetzung nach irgendeinem der vorstehenden Ansprüche zur Verwendung als Medikament.

4. Tablette mit verzögerter Freisetzung nach Anspruch 3 zur Verwendung bei der Behandlung und/oder Vorbeugung von Angina pectoris und/oder koronarer Arteriosklerose, wo die Tablette optional einmal pro Tag verabreicht wird.

5. Verpackung umfassend zwischen 28 und 62 Tabletten mit verzögerter Freisetzung nach den Ansprüchen 1 und 2, wobei die Tabletten mit verzögerter Freisetzung in mindestens einer Blisterverpackung konditioniert sind, die die Tabletten mit verzögerter Freisetzung vor Licht schützt, wobei die Blisterverpackung selbst in der Verpackung verpackt ist.

6. Verfahren zur Herstellung einer Tablette mit verzögerter Freisetzung nach irgendeinem der Ansprüche 1 und 2, umfassend:
- Bereitstellen eines im Wesentlichen trockenen Vorgemisches, umfassend einen oder mehrere pharmazeutisch annehmbare Hilfsstoffe,
- Mischen von Molsidomin in im Wesentlichen trockener Form mit dem im Wesentlichen trockenen Vorgemisch um eine im Wesentlichen trockene Zubereitung zu erhalten, und
- Komprimieren der im Wesentlichen trockenen Zubereitung, um die Tablette mit verzögerter Freisetzung zu erhalten.

7. Verfahren nach Anspruch 6, umfassend:
- Bereitstellen eines im Wesentlichen trockenen Vorgemisches, umfassend Calcium-Dihydrat-Monohydrogenphosphat, mikrokristalline Cellulose und wasserfreie kolloidale Kieselsäure,
- Mischen von Molsidomin in im Wesentlichen trockener Form und eines lipophilen Matrixmittels mit dem im Wesentlichen trockenen Vorgemisch, um eine im Wesentlichen trockene Zubereitung zu erhalten,
- Komprimieren der im Wesentlichen trockenen Zubereitung, um die Tablette mit verzögerter Freisetzung zu erhalten,
- und optional Konditionieren der Tablette mit verzögerter Freisetzung, vorzugsweise in einer Blisterverpackung, welche die Tabletten mit verzögerter Freisetzung vor Licht schützt.

## Claims

1. A sustained release tablet comprising:
- 14.00 to 24.00 mg of molsidomine,
- 45.00 to 80.00 mg of calcium monohydrogen phosphate dihydrate,
- 35.00 to 60.00 mg of a lipophilic matrix agent,
- 15.00 to 45.00 mg of microcrystalline cellulose,
- 0.10 to 10.00 mg of anhydrous colloidal silica, and
- 0.50 to 5.00 mg of magnesium stearate,
wherein said tablet is obtained by a direct compression method.

2. The sustained release tablet of claim 1, wherein said tablet has an *in vitro* release rate such that between 40 and 60 % by weight of the molsidomine is released after 6 hours and less than 80 % by weight of the molsidomine is released after 10 hours.

3. The sustained release tablet according to any one of the preceding claims for use as a medicament.

4. The sustained release tablet according to claim 3 for use in the treatment and/or prevention of the angina pectoris and/or the coronary atherosclerosis, optionally in which the tablet is administered once daily.

5. A packaging comprising between 28 and 62 sustained release tablets according to claims 1 and 2, said sustained release tablets being packaged in at least one blister protecting said sustained release tablets from light, said blister itself being packaged in said package.

6. A method of producing a sustained release tablet according to any one of claims 1 and 2 comprising:
- providing a substantially dry premix comprising one or more pharmaceutically acceptable excipients,
- mixing molsidomine in substantially dry form with said substantially dry premix to obtain a substantially dry preparation, and
- compressing said substantially dry preparation so as to obtain said sustained release tablet.

7. The method according to claim 6, comprising:
- providing a substantially dry premix comprising calcium monohydrogen phosphate dihydrate, microcrystalline cellulose and anhydrous colloidal silica,
- mixing molsidomine in substantially dry form and a lipophilic matrix agent with said substantially dry premix to obtain a substantially dry preparation,
- compressing said substantially dry preparation so as to obtain said sustained release tablet,
- and optionally conditioning said sustained release tablet, preferably in a blister protecting said sustained release tablets from light.
